(19) Europäisches Patentamt
European Patent Office
Office européen des brevets

(11) Numéro de publication: **0 135 410**
**B1**

(12) # FASCICULE DE BREVET EUROPÉEN

(45) Date de publication du fascicule du brevet: **21.09.88**

(21) Numéro de dépôt: **84401480.3**

(22) Date de dépôt: **12.07.84**

(51) Int. Cl.⁴: **C 07 D 498/18**, A 61 K 31/44 // (C07D498/18, 273:00, 263:00, 209:00),(A61K31/44, 31:42)

(54) **Dérivés de synergistines, leur préparation et les compositions pharmaceutiques qui les contiennent.**

(30) Priorité: **13.07.83 FR 8311707**

(43) Date de publication de la demande: **27.03.85 Bulletin 85/13**

(45) Mention de la délivrance du brevet: **21.09.88 Bulletin 88/38**

(84) Etats contractants désignés: **AT BE CH DE FR GB IT LI LU NL SE**

(56) Documents cité:
**BULLETIN DE LA SOCIETE CHIMIQUE DE FRANCE, no. 2, 1969, pages 585-591, Paris, FR; J.PREUD'HOMME et al.: "Pristinamycine isolement, caractérisation et identification des constituants"**
**CHEMICAL ABSTRACTS, vol. 71, 1969, pages 201-202, no. 47912e, Columbus, Ohio, USA; M. DELEPINE: "Pristinamycin; an antibiotic with two synergystic components"**

(73) Titulaire: **RHONE- POULENC SANTE, Les Miroirs 18 Avenue d'Alsace, F-92400 Courbevoie Cédex (FR)**

(72) Inventeur: **Corbet, Jean- Pierre, Les Marronniers Résidence "Charrière Blanche", F-69140 Ecully (FR)**
Inventeur: **Cotrel, Claude, 17A avenue du Docteur Arnold Netter, F-75012 Paris (FR)**
Inventeur: **Farge, Daniel, 30 rue des Pins Sylvestres, F-94320 Thiais (FR)**
Inventeur: **Paris, Jean- Marc, 8 rue des Acacias, F-77360 Vaires Sur Marne (FR)**

(74) Mandataire: **Gaumont, Robert, RHONE- POULENC RECHERCHES Service Brevets Pharma 25, Quai Paul Doumer, F-92408 Courbevoie Cedex (FR)**

EP 0 135 410 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

## Description

La présente invention concerne de nouveaux dérivés de la pristinamycine $II_b$ de formule générale I:

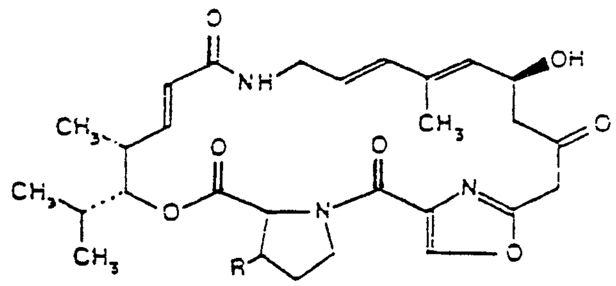

(I)

leurs sels, leur préparation et les compositions pharmaceutiques qui les contiennent.

Dans la formule générale I, R représente

- soit un radical alcoylthio substitué:
- i) par un ou deux radicaux alcoylamino ou dialcoylamino dont les parties alcoyle peuvent éventuellement former ensemble avec l'atome d'azote auquel elles sont liées un hétérocycle saturé choisi parmi pyrrolidinyle-1, pipéridino, azétidinyle-1, azépinyle-1, morpholino, thiomorpholino et pipérazinyle-1 (éventuellement substitué par un radical alcoyle), ou bien
- ii) par un radical pyrrolidinyle-2 ou 3, pipéridyle-2, 3 ou 4, azétidinyle-2 ou 3 ou azépinyle-2, 3 ou 4,
- soit un radical de formule générale II:

Het-S- (II)

dans laquelle Het représente un radical pyrrolidinyle-3, pipéridyle-3 ou 4, azétidinyle-3 ou azépinyle-3 ou 4, (éventuellement substitués sur l'atome d'azote par un radical alcoyle)

- soit un radical dialcoylamino dont les parties alcoyle peuvent éventuellement former ensemble avec l'atome d'azote auquel elles sont liées un hétérocycle saturé choisi parmi pyrrolidinyle-1, pipéridino, azétidinyle-1, azépinyle-1, morpholino, thiomorpholino et pipérazinyle-1 (éventuellement substitué par un radical alcoyle), étant entendu que les radicaux alcoyle et portions alcoyle cités ci-dessus ou qui seront cités par la suite contiennent 1 à 5 atomes de carbone en chaîne droite ou ramifiée.

Il est entendu que les produits de formule générale I peuvent présenter des formes isomères et que ces isomères et leurs mélanges entrent dans le cadre de la présente invention.

Selon la présente invention, les produits de formule générale I peuvent être préparés par action d'un produit de formule générale III:

R-H (III)

dans laquelle R est défini comme précédemment sur le produit de formule IV:

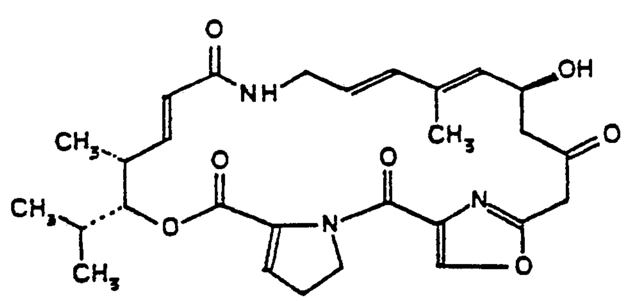

(IV)

c'est-à-dire la pristinamycine $II_A$.

La réaction s'effectue généralement sans solvant ou dans un solvant organique tel qu'un alcool comme le méthanol ou l'éthanol ou un solvant chloré comme le chlorure de méthylène, le dichloro-1,2 éthane ou le chloroforme, ou dans un mélange de ces solvants (par exemple chlorure de méthylène-méthanol) à une température comprise entre 0 et 50° C.

Il peut être parfois avantageux d'opérer en présence d'une amine tertiaire, par exemple la triéthylamine, ou d'une éthanolamine (diméthyléthanolamine par exemple).

Il est entendu pour l'homme du métier que, lorsque R représente un radical qui contient une fonction amine secondaire pouvant interférer avec la réaction, cette dernière fonction devra être préalablement protégée avant de faire réagir le produit de formule générale III sur le produit de formule IV. On peut utiliser à cet effet tout moyen habituel permettant de bloquer une fonction amine secondaire sous forme d'un radical labile qui

pourra être éliminé après réaction du produit de formule générale III sur le produit de formule IV. Il est particulièrement avantageux d'utiliser comme radical bloquant le radical trifluoracétyle. Celui-ci pourra ensuite être éliminé à l'aide d'une solution aqueuse de bicarbonate alcalin tel que le bicarbonate de sodium ou de potassium.

Les nouveaux produits de formule générale I peuvent être purifiés par les méthodes connues habituelles, par exemple cristallisation, chromatographie ou extractions successives en milieu acide et basique. Pour l'homme du métier connaissant la sensibilité des synergistines en milieu alcalin, on entend par "milieu basique" un milieu juste suffisamment alcalin pour libérer la substance-mère de son sel d'addition avec un acide, c'est-à-dire un milieu dont le pH n'excède pas 7,5 à 8.

Il est bien connu que les synergistines obtenues par fermentation constituent des produits très recherchés par les médecins pour le traitement de beaucoup d'affections dues à des bactéries Gram-positives (du genre Staphylocoques, Streptocoques, pneumocoques, entérocoques) et Gram-négatives (du genre Haemophilus, gonocoques, méningocoques). Toutefois ces produits présentent l'inconvénient d'être insolubles en milieu aqueux et ne peuvent donc être administrés que par voie orale, généralement sous forme de gellules, de dragées ou de comprimés. Compte tenu de cette insolubilité, il est impossible d'utiliser les synergistines connues jusqu'ici lorsque le malade n'est pas en l'état de déglutir; c'est notamment le cas en pédiatrie et en réanimation, alors que le spectre d'activité de ces produits en ferait une indication précieuse dans un grand nombre de circonstances, par exemple dans les cas de septicémies comateuses.

Les nouveaux produits selon l'invention présentent l'avantage considérable de pouvoir être solubilisés dans l'eau, généralement à l'état de sels, aux doses thérapeutiques utilisables et d'exalter par un phénomène de synergie l'action antibactérienne de la pristinamycine $I_A$, de la virginiamycine S ou de dérivés de synergistines solubles de formule générale V:

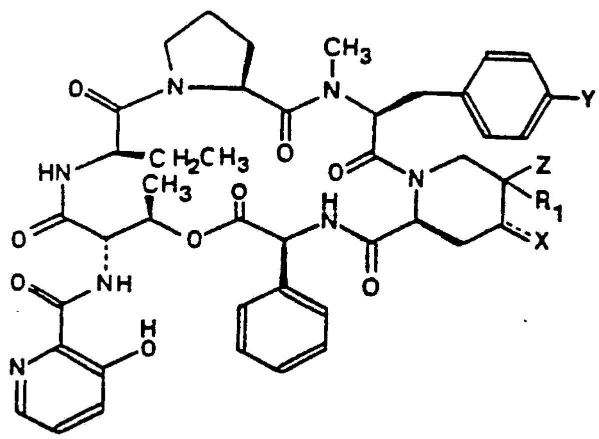

(V)

dans laquelle Y représente un atome d'hydrogène ou un radical diméthylamino et

1) ou bien ----- représente une liaison simple, Z et $R_1$ représentent un atome d'hydrogène et X représente un radical de formule générale VI:

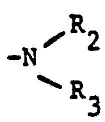

(VI)

dans laquelle:

- soit $R_2$ représente un atome d'hydrogène et $R_3$ représente un radical hydroxy ou alcoyle éventuellement substitué par un radical carboxy, alcoyloxycarbonyle, hydroxy, alcoylamino ou dialcoylamino dont les radicaux alcoyle peuvent former avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle ou azépinyle, ou bien $R_3$ représente un radical cycloalcoyle contenant 3 à 7 atomes de carbone ou un hétérocycle saturé à 4 à 7 chaînons choisi parmi les cycles azétidine, pyrrolidine, pipéridine et azépine, ces hétérocycles pouvant être éventuellement substitués sur l'atome d'azote par un radical alcoyle,

- soit $R_2$ représente un radical formyle ou alcoylcarbonyle et $R_3$ représente un radical alcoyle substitué par un radical carboxy, alcoylamino ou dialcoylamino dont les radicaux alcoyle peuvent former avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle ou azépinylé, ou bien $R_3$ représente un hétérocycle à 4 à 7 chaînons choisi parmi les cycles azétidine, pyrrolidine, pipéridine et azépine, ces hétérocycles pouvant être substitués sur l'atome d'azote par un radical alcoyle,

- soit $R_2$ et $R_3$ identiques ou différents, représentent un radical alcoyle éventuellement substitué par un radical carboxy, alcoyloxycarbonyle, hydroxy, alcoylamino ou dialcoylamino dont les radicaux alcoyle forment

éventuellement avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle ou azépinyle

- soit $R_2$ et $R_3$ forment ensemble avec l'atome d'azote auquel ils sont rattachés un hétérocycle à 4 à 7 chaînons choisi parmi les cycles azétidine, pyrrolidine, pipéridine, morpholine et pipérazine éventuellement substitué par un radical alcoyle,

2) ou bien ----- représente une double liaison, X représente un atome d'oxygène et Z représente un radical de formule générale VII:

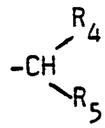

(VII)

défini de la manière suivante:

a) soit $R_1$ et $R_5$ représentent chacun un atome d'hydrogène et $R_4$ représente un radical pyrrolidinyl-3 thio ou pipéridyl-3 ou 4 thio (ces radicaux étant éventuellement substitués par un radical alcoyle) ou bien $R_4$ représente un radical alcoylthio substitué par un ou deux radicaux hydroxysulfonyle, alcoylamino, dialcoylamino (éventuellement substitué par un radical mercapto ou dialcoylamino) ou par un ou deux cycles choisis parmi pipérazino (éventuellement substitué par un radical alcoyle ou mercaptoalcoyle), morpholino, thiomorpholino, pipéridino, pyrrolidinyle-1, pipéridyle-2, 3 ou 4 et pyrrolidinyle-2 ou 3 (ces deux derniers cycles étant éventuellement substitués sur l'atome d'azote par un radical alcoyle),

b) soit $R_1$ et $R_5$ forment ensemble une liaison de valence et $R_4$ représente un radical pyrrolidinyl-3 amino, pipéridyl-3 ou 4 amino, pyrrolidinyl-3 oxy, pipéridyl-3 ou 4 oxy, pyrrolidinyl-3 thio, pipéridyl-3 ou 4 thio (ces radicaux étant éventuellement substitués sur l'atome d'azote du cycle par un radical alcoyle), ou bien $R_4$ représente un radical alcoylamino, alcoyloxy ou alcoylthio substitués par un ou deux radicaux hydroxysulfonyle, alcoylamino, dialcoylamino (éventuellement substitué par un radical dialcoylamino), trialcoylammonio ou imidazolyle-4 ou 5 ou par un ou deux cycles choisis parmi pipérazino (éventuellement substitué par un radical alcoyle ou mercaptoalcoyle), morpholino, thiomorpholino, pipéridino, pyrroli-dinyle-1, pipéridyle-2, 3 ou 4 et pyrrolidinyle-2 ou 3 (ces deux derniers cycles étant éventuellement substitués sur l'atome d'azote par un radical alcoyle), étant entendu que les radicaux alcoyle et portions alcoyle se rapportant aux symboles de la formule générale V contiennent 1 à 5 atomes de carbone et sont en chaîne droite ou ramifiée.

Certains des dérivés de synergistines de formule générale V peuvent présenter des formes isomères. Il est entendu que ces formes isomères aussi bien que leurs mélanges peuvent être avantageusement associés aux produits de formule générale I.

Les produits de formule générale V sont décrits plus en détail dans le brevet européen 0 133 097.

Les produits de formule générale V définis comme précédemment en 1) à l'exception de ceux pour lesquels $R_2$ représente un radical formyle ou alcoylcarbonyle, peuvent être préparés par action d'une amine de formule générale VIII:

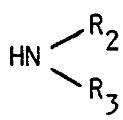

(VIII)

dans laquelle $R_2$ et $R_3$ sont définis comme ci-dessus, sur une synergistine de formule générale:

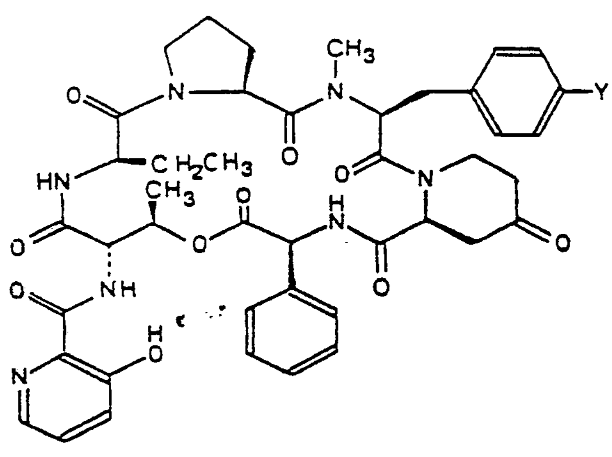

(IX)

dans laquelle Y représente un atome d'hydrogène (virginiamycine S) ou le radical diméthylamino (pristinamycine $I_A$), en présence d'un cyanoborohydrure alcalin.

On opère généralement avec un excès d'amine de formule générale VIII en présence d'un cyanoborohydrure alcalin comme le cyanoborohydrure de sodium, dans un solvant organique tel qu'un alcool dans lequel on a dissout de l'acide chlorhydrique gazeux (méthanol chlorhydrique ou éthanol chlorhydrique), à une température comprise entre 0°C et la température de reflux du mélange réactionnel, de préférence à une température voisine de 20°C.

La réaction peut être avantageusement effectuée en présence d'un agent de dessication tel que des tamis moléculaires.

Les produits de formule générale V définis comme précédemment en 1) dans laquelle $R_2$ représente un radical formyle ou alcoylcarbonyle et $R_3$ représente un radical alcoyle substitué par un radical carboxy, alcoylamino ou dialcoylamino dont les radicaux alcoyle forment éventuellement avec l'atome d'azote auquel ils sont rattachés, un hétérocycle à 4 à 7 chaînons choisis parmi azétidinyle, pyrrolidinyle, pipéridinyle, pipérazinyle, alcoylpipérazinyle ou azépinyle, ou représente un hétérocycle saturé à 4 à 7 chaînons choisi parmi les cycles azétidine, pyrrolidine, pipéridine et azépine, ces hétérocycles pouvant être substitués sur l'atome d'azote par un radical alcoyle, et Y est défini comme précédemment, peuvent être préparés par action d'un produit de formule générale X:

$R_6$ - CO - Q (X)

dans laquelle $R_6$ représente un atome d'hydrogène ou un radical alcoyle et Q réprésente un atome d'halogène ou un radical alcoilcarbonyloxy, sur un produit de formule générale XI:

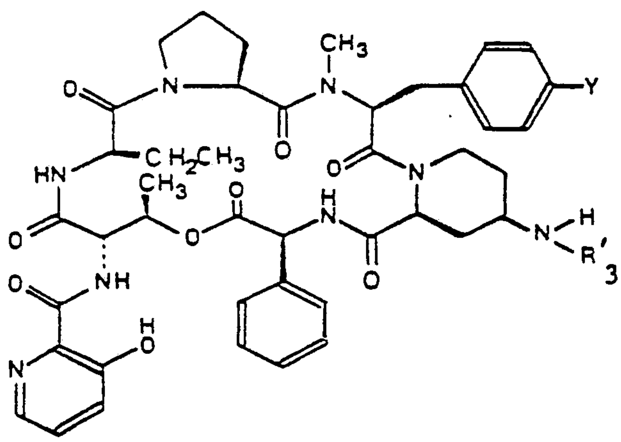

(XI)

dans laquelle Y est défini comme précédemment et $R'_3$ a la définition de $R_3$ correspondante donnée ci-dessus.

La réaction s'effectue généralement dans un solvant organique tel que la pyridine, dans un solvant chloré (chlorure de méthylène) ou un éther (tétrahydrofuranne) en présence d'un accepteur d'acide tel qu'une base organique comme la triéthylamine ou une base minérale telle qu'un carbonate ou un bicarbonate alcalin comme le bicarbonate de sodium en opérant à une température comprise entre 0°C et 80°C.

Il est entendu pour l'homme du métier que, lorsque $R'_3$ représente un radical contenant une fonction amine secondaire, ladite fonction doit être protégée avant de faire réagir le produit de formule générale X sur le produit de formule générale XI. On utilise à cet effet tout moyen de blocage habituel employé pour protéger une fonction amine et pouvant être éliminé par la suite, sans toucher au reste de la molécule. Il est particulièrement avantageux d'utiliser comme radical bloquant le radical trifluoracétyle; celui-ci peut ensuite être éliminé à l'aide d'une solution aqueuse de bicarbonate alcalin tel que le bicarbonate de sodium ou de potassium.

Il est également entendu pour l'homme du métier que, lorsque dans la formule générale VIII $R_2$ et/ou $R_3$ représentent un radical contenant une fonction amine secondaire, cette dernière doit être préalablement protégée avant de faire réagir le produit de formule générale VIII sur le produit de formule générale IX. Le blocage et le déblocage s'effectuent comme décrit précédemment.

Les produits de formule générale V définis comme précédemment en 2) dans laquelle Y est défini comme précédemment et les autres symboles sont définis comme précédemment en 2)a) peuvent être préparés par action d'un produit de formule générale XII:

$R'_4$-H (XII)

dans laquelle R'$_4$ a la définition de $R_4$ donnée précédemment en 2)a) sur un produit de formule générale XIII:

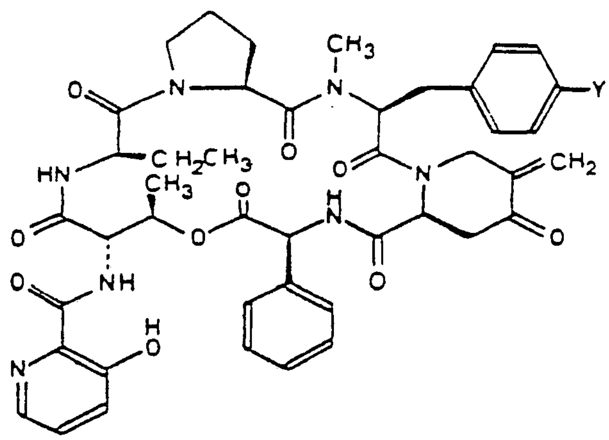

(XIII)

dans laquelle Y est défini comme précédemment.

On opère généralement dans un solvant organique tel qu'un alcool comme le méthanol ou un solvant chloré comme le chloroforme ou un mélange de ces solvants, à une température comprise entre 0° C et la température de reflux du mélange réactionnel, de préférence à une température voisine de 20° C.

Les produits de formule générale XIII peuvent être préparés par action d'un borohydrure alcalin tel que le cyanoborohydrure de sodium sur un produit de formule générale XIV:

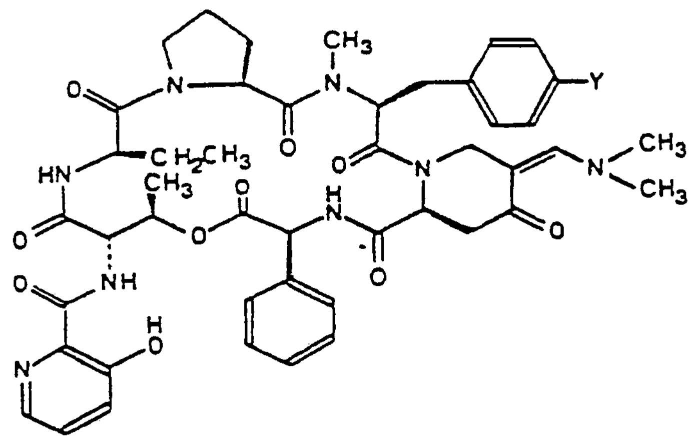

(XIV)

dans laquelle Y est défini comme précédemment.

On opère généralement dans un solvant organique tel qu'un éther comme le tétrahydrofuranne ou un alcool par exemple l'isopropanol en présence d'un acide tel que l'acide trifluoroacétique, à une température comprise entre 0° C et la température de reflux du mélange réactionnel, de préférence à une température voisine de 20° C.

Les produits de formule générale XIV peuvent être obtenus par action d'un produit de formule XV:

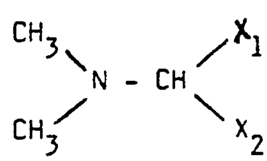

(XV)

dans laquelle ou bien $X_1$ représente un radical alcoyloxy et $X_2$ représente un radical alcoyloxy ou diméthylamino, ou bien $X_1$ et $X_2$ représentent tous les deux un radical diméthylamino, sur un produit de formule générale IX.

Dans la pratique, il est avantageux de faire réagir le tert-butoxy bis(diméthylamino) méthane sur le produit de formule générale IX, en opérant dans un solvant organique tel qu'un solvant chloré comme le dichloro-1,2 éthane ou un amide (diméthylformamide par exemple) à une température comprise entre 0° C et 80° C, de préférence à une température voisine de 20° C.

Les produits de formule générale XV peuvent être préparés selon les méthodes décrites par H. BREDERECK et coll., Chem. Ber., 101, 41 et 3058 (1968) et Chem. Ber., 106, 3725 (1973).

Les produits de formule générale V dans laquelle Y est défini comme précédemment et les autres symboles sont définis comme précédemment en 2) b) à l'exception pour $R_4$ de représenter un radical pyrrolidinyl-3 oxy, pipéridyl-3 ou 4 oxy ou alcoyloxy éventuellement substitués comme défini en 2) b), peuvent être préparés par action d'un produit de formule générale XVI:

$R''_4$ - H (XVI)

dans laquelle $R''_4$ a la définition de $R_4$ donnée ci-dessus, sur un produit de formule générale XIV dans laquelle Y est défini comme précédemment.

La réaction s'effectue en milieu organique, en présence d'un acide (par exemple l'acide acétique ou un mélange d'acide acétique et de quantités catalytiques d'acide trifluoracétique), en présence ou non d'un solvant, à une température comprise entre 0 et 50°C; de préférence à une température voisine de 20°C.

Le cas échéant, le solvant peut être choisi parmi les solvants organiques comme les éthers (tétrahydrofuranne), les alcools (éthanol) et les solvants chlorés (chlorure de méthylène ou chloroforme par exemple).

Les produits de formule générale V dans laquelle Y est défini comme précédemment et les autres symboles sont définis comme précédemment en 2) b) peuvent être préparés par action d'un produit de formule générale XVII:

$R'''_4$-H (XVII)

dans laquelle $R'''_4$ est défini comme $R_4$ en 2) b), sur un produit de formule générale XVIII:

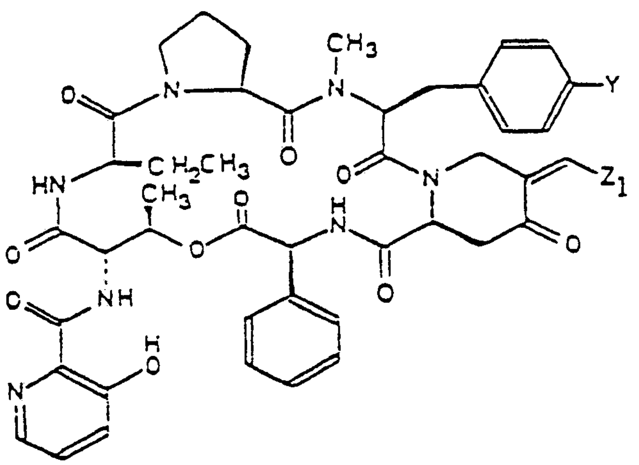

(XVIII)

dans laquelle Y est défini comme précédemment et $Z_1$ représente un radical tosyloxy, acétyloxy, triméthylsilyloxy ou dialcoyloxyphosphortloxy dont les parties alcoyle contiennent 1 à 4 atomes de carbone en chaîne droite ou ramifiée, ou bien $Z_1$ représente un atome de chlore.

On opère généralement dans un solvant organique tel qu'un éther comme le tétrahydrofuranne, un alcool comme l'éthanol ou un solvant chloré (chlorure de méthylène ou chloroforme par exemple) à une température voisine de 20°C. La réaction s'effectue en milieu basique, par exemple en présence d'un hydrure alcalin ou d'un alcoolate alcalin comme l'éthylate de sodium ou le tert-butylate de potassium.

Lorsque $R'''_4$ est autre qu'alcoyloxy substitué ou hétérocyclyloxy, il est également possible d'opérer soit en milieu neutre à une température comprise entre 0 et 50°C, dans l'un des solvants cités ci-dessus, soit en milieu acide dans des conditions identiques à celles décrites précédemment pour l'action d'un produit de formule générale XVI sur un produit de formule générale XIV.

Les produits de formule générale XVIII peuvent être préparés par hydrolyse acide d'un produit de formule générale XIV pour obtenir un produit de formule générale XIX:

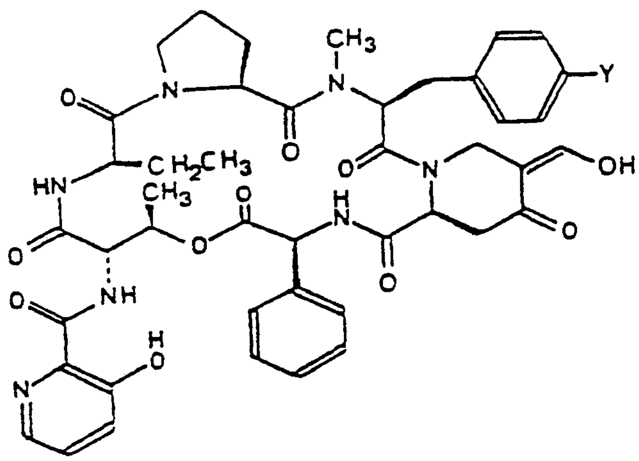

(XIX)

suivie:

α) ou bien de l'action d'un produit de formule générale XX:

$Z'_1$-X (XX)

dans laquelle X représente un atome d'halogène et $Z'_1$ a la définition donnée précédemment pour $Z_1$ à l'exception de représenter un atome de chlore

β) ou bien de l'action d'un produit de formule XXI:

$(C_6H_5)_3$ P. $Cl_2$ (XXI)

pour obtenir un produit de formule générale XVIII dans laquelle $Z_1$ représente un atome de chlore.

L'hydrolyse du produit de formule générale XIV en produit de formule générale XVIII s'effectue au moyen d'une solution aqueuse d'un acide minéral tel qu'une solution aqueuse 0,1N d'acide chlorhydrique en opérant à une température voisine de 20°C.

La réaction du produit de formule générale XX sur le produit de formule générale XIX s'effectue généralement dans un solvant organique tel que le chlorure de méthylène en présence d'un accepteur d'acide tel qu'une base organique comme la triéthylamine ou une base minérale comme un carbonate ou un bicarbonate alcalin, par exemple le bicarbonate de sodium ou de potassium. On opère généralement à une température comprise entre -20 et +20°C.

La réaction du produit de formule générale XXI sur le produit de formule générale XIX s'effectue généralement dans un solvant chloré tel que le chlorure de méthylène à une température comprise entre -20 et +20°C.

Les produits de formules générales III, VIII, XII, XVI et XVII peuvent être préparés selon ou par analogie avec les méthodes décrites ci-après dans les exemples et notamment selon:

- G.G. Urquart et coll., Org. Synth., 21, 36 (1941)
- A.I. Vogel, J. Chem. Soc., 1822 (1948)
- J.H. Chapman et L.N. Owen, J. Chem. Soc., 579 (1950)
- H.R. Snyder et coll., J. Am. Chem. Soc., 69, 2672 (1947)
- D.D. Reynolds et coll., J. Org. Chem., 26, 5125 (1961)
- J.W. Haeffele et coll., Proc. Sci. Toilet Goods Assoc., 32, 52 (1959)
- H. Barrer et coll., J. Org. Chem., 27, 641 (1962)
- J.H. Biel et coll., J. Amer. Chem. Soc., 77, 2250 (1955) lorsqu'il s'agit d'un produit de formule générale III, XII, XVI ou XVII pour lequel R, $R'_4$, $R''_4$ ou $R'''_4$ représente un radical alcoylthio substitué ou hétérocyclylthio, ou selon:
- A.J.W. Headlee et coll., J. Amer. Chem. Soc., 55, 1066 (1933)
- B.K. Campbell et K.N. Campbell, J. Amer. Chem. Soc., 60, 1372 (1938)
- R.C. Elderfield et coll., J. Amer. Chem. Soc., 68, 1579 (1946)

lorsqu'il s'agit d'un produit de formule générale XVI ou XVII pour lequel $R''_4$ ou $R'''_4$ représente un radical alcoyloxy substitué ou hétérocyclyloxy, ou selon:

- J. Amer. Chem. Soc., 54, 1499 (1932) et
- J. Amer. Chem. Soc., 54, 3441 (1932),

lorsqu'il s'agit d'un produit de formule générale VIII ou de formule générale III, XVI ou XVII pour lequel R, $R''_4$ ou $R'''_4$ sont des radicaux alcoylamino substitués, ou selon:

- E.F. Elslager et coll., J. Med. Chem., 17, 99 (1974)
- L.M. Werbel et coll., J. Het. Chem., 10, 363 (1973),

lorsqu'il s'agit d'un produit de formule générale III, XVI ou XVII pour lequel R, R"$_4$ ou R'''$_4$ sont des radicaux hétérocyclylamino.

Il est entendu que dans les méthodes ci-dessus, lorsque $R_2$, $R_3$, R'$_4$, R"$_4$ ou R'''$_4$ contiennent un radical alcoylamino pouvant interférer avec la réaction, ce dernier est préalablement protégé par toute méthode connue qui n'altère pas le reste de la molécule.

De même lorsque les radicaux R'$_4$, R"$_4$ et R'''$_4$ dans les produits de formules générales XII, XVI, et XVII contiennent une fonction amine secondaire pouvant interférer avec la réaction, celle-ci doit être protégée avant de faire réagir les produits correspondants respectivement avec les produits de formules générales XIII, XIV et XVIII. Le radical protecteur est éliminé après réaction. On opère à cet effet dans les conditions décrites précédemment pour le radical R du réactif de formule générale III.

Le cas échéant, les isomères des produits de formule générale I et/ou des produits de formule générale V peuvent être séparés par chromatographie ou par chromatographie liquide hautes performances.

Les produits de formule générale V peuvent être purifiés comme mentionné précédemment pour les produits de formule générale I.

Au laboratoire, les produits de formule générale I syngergisent l'action anti-microbienne des produits de formule générale V à des doses comprises entre 5 et 200 mg/kg chez la souris par voie sous-cutanée, notamment dans la septicémie provoquée par Staphylococcus aureus Smith lorsqu'ils sont mélangés aux produits de formule générale V dans des proportions variant entre 10 - 90 % et 90 - 10 %.

La toxicité aigüe des produits de formule générale I, exprimée par la $DL_{50}$, est généralement comprise entre 300 et 900 mg/kg par voie sous-cutanée chez la souris.

D'un intérêt particulier pour leur bon effet synergisant vis-à-vis de la pristinamycine $I_A$, sont les produits de formule générale I dans laquelle R représente un radical alcoylthio substitué par un ou deux radicaux dialcoylamino dont les parties alcoyle peuvent éventuellement former avec l'atome d'azote auquel elles sont rattachées, un hétérocycle saturé choisi parmi pyrrolidinyle-1 et pipérazinyle-1 éventuellement substitué par un radical alcoyle, ou bien R représente soit un radical de formule générale II dans laquelle Het représente un radical pipéridyle-4 éventuellement substitué sur l'atome d'azote par un radical alcoyle, soit un radical dialcoylamino dont les parties alcoyle peuvent former avec l'atome d'azote auquel elles sont liées, un cycle pipérazinyle-1 éventuellement substitué par un radical alcoyle, étant entendu que les radicaux et portions alcoyles cités ci-dessus sont droits ou ramifiés et contiennent 1 à 3 atomes de carbone;

parmi ces produits plus spécialemnt intéressants sont les produits de formule générale I dans laquelle R est un radical alcoylthio ramifié contenant 1 à 3 atomes de carbone substitué par un radical dialcoylamino ou représente un cycle alcoyl-4 pipérazinyle-1, étant entendu que sauf mention spéciale les radicaux alcoyle contiennent 1 ou 2 atomes de carbone; et notamment les produits suivants:

- (diéthylamino-1 propyl-2) thio-26 pristinamycine $II_B$
- (méthyl-4 pipérazinyl-1)-26 pristinamycine $II_B$.

Pour l'emploi thérapeutique, il peut être fait usage des nouveaux produits selon l'invention tels quels c'est-à-dire à l'état de base en association avec des synergistines déjà connues, mais comme le principal avantage des produits selon l'invention est leur solubilisation possible dans l'eau, il est particulièrement avantageux de les utiliser sous forme de sels pharmaceutiquement acceptables, en association avec des synergistines connues ou les synergistines de formule générale V elles-mêmes solubilisées soit à l'état de sels pharmaceutiquement acceptables soit, le cas échéant, à l'état de base lorsque la solubilité est suffisante pour que la solution obtenue contienne une quantité de produit au moins égale à la dose thérapeutiquement active.

Comme sels pharmaceutiquement acceptables, aussi bien pour les produits de formule générale I que pour les produits de formule générale V, on peut citer les sels d'addition avec les acides minéraux tels que chlorhydrates, bromhydrates, sulfates, nitrates, phosphates, ou organiques tels que acétates, propionates, succinates, maléates, fumarates, méthanesulfonates, p.toluène-sulfonates, iséthionates ou des dérivés de substitution de ces composés. Comme sels pharmaceutiquement acceptables, on peut encore citer les sels avec les métaux alcalins tels que les sels de sodium, de potassium, de lithium, avec les métaux alcalino-terreux tels que le sel de magnésium, le sel d'ammonium et les sels d'addition avec les bases organiques azotées: éthanolamine, diéthanolamine, triméthylamine, triéthylamine, méthylamine, propylamine, diisopropylamine, N,N-diméthyléthanolamine, benzylamine, dibenzylamine, dicyclohexylbenzylamine, N-benzyl-β-phénéthylamine, N,N'-dibenzyléthylènediamine, benzhydrylamine, arginine, leucine, lysine ou N-méthylglucamine.

Comme sels pharmaceutiquement acceptables pour les produits de formule générale V dans laquelle Z représente un radical de formule générale VII dans laquelle $R_4$ représente un radical trialcoylammonio, on peut citer les sels d'ammonium quaternaire correspondant aux anions énumérés ci-dessus.

Les exemples suivants, donnés à titre non limitatif, montrent comment l'invention peut être mise en pratique. Les spectres de RMN des produits illustrés dans ces exemples présentent des caractéristiques générales qui sont communes à tous les produits de formule générale I ou de formule générale V et des caractéristiques particulières qui sont propres à chacun des produits en fonction des substituants. Dans l'exemple 1, il est donné l'attribution de tous les protons de la molécule; dans les exemples ne sont mentionnées que les caractéristiques particulières dues aux radicaux variables. Pour les produits de formule générale I tous les protons sont désignés selon la numérotation indiquée dans la formule XXII:

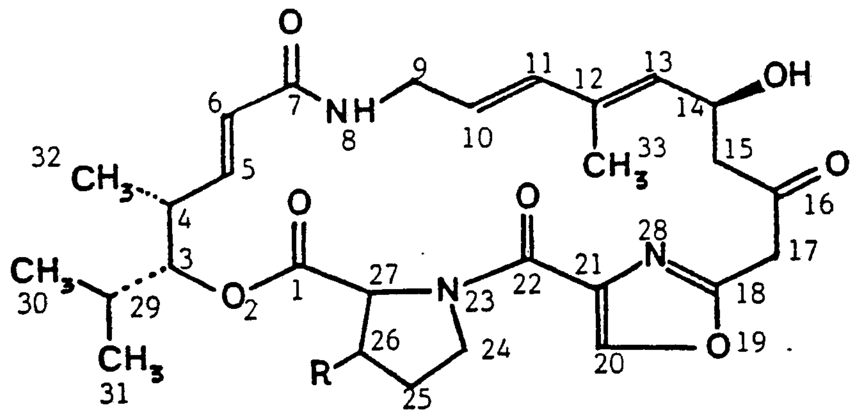

(XXII)

Pour les synergistines de formule générale V tous les protons sont désignés selon la numérotation indiquée dans la formule générale XXIII; cette numérotation est celle recommandée par J.O. ANTEUNIS et coll., [Eur. J. Biochem., 58, 259 (1975)].

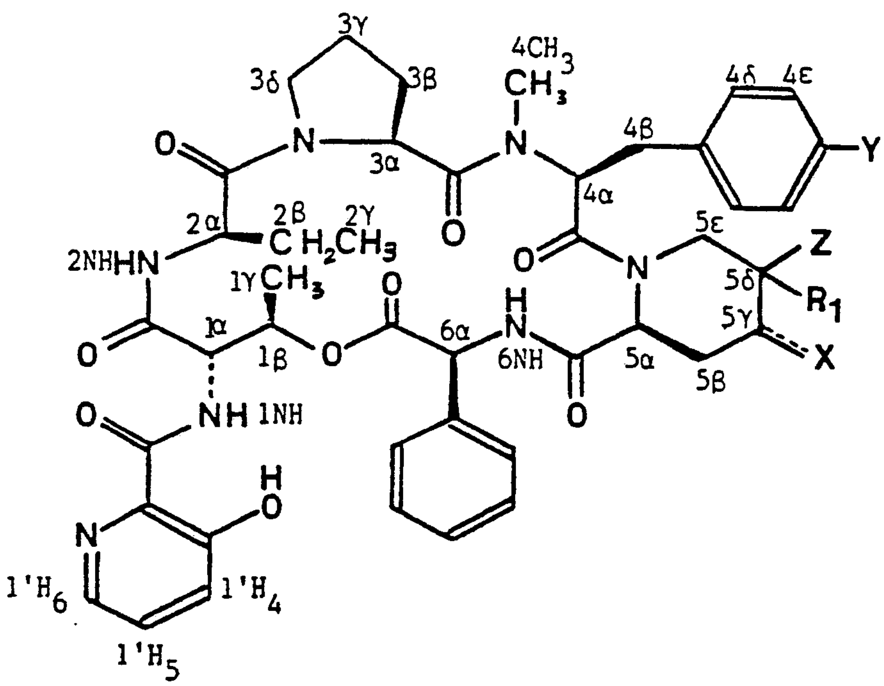

(XXIII)

Tous les spectres ont été faits à 250 MHz dans le deutérochloroforme; les déplacements chimiques sont exprimés en ppm par rapport au signal du tétraméthylsilane. Les abréviations utilisées dans la suite sont les suivantes:

s = singulet
d = doublet
t = triplet
mt = multiplet
m = massif
dd = doublet de doublet
dt = doublet de triplet
ddd = doublet de doublet de doublet
dddd = doublet de doublet de doublet de doublet

Dans les exemples qui suivent, on appelle chromatographie "flash" une technique de purification caractérisée en ce qu'on utilise une colonne de chromatographie courte et qu'on opère sous une pression moyenne (50 kPa) en utilisant une silice de granulométrie 40 - 53 µm, selon W.C. STILL, M. KAHN et A. MITRA. [J. Org. Chem., 43, 2923 (1978)].

**Exemple 1**

A une suspension de 13,1 g de pristinamycine $II_A$ dans 150 $cm^3$ de méthanol, on ajoute une solution de 3,7 g de diéthylamino éthanethiol dans 15 $cm^3$ de chlorure de méthylène. La solution obtenue est agitée pendant 18 heures à une température voisine de 20° C, puis versée dans 1500 $cm^3$ d'eau distillée; le mélange obtenu est extrait 3 fois par 1000 $cm^3$ au total de chlorure de méthylène. Les phases organiques sont réunies, séchées sur du sulfate de magnésium, filtrées puis concentrées à sec sous pression réduite (2,7 kPa) à 30° C. Le résidu obtenu est purifié par chromatographie "flash" [éluant: chloroforme-méthanol (90 - 10 en volumes]; après concentration à sec des fractions 5 à 23 sous pression réduite (2,7 kPa) à 30°C, on obtient 12,4 g de (diéthylamino-2 éthyl) thio-26 pristinamycine $II_B$ sous forme d'une poudre jaune fondant vers 105° C.

| δ (ppm) | Forme | Attribution |
|---|---|---|
| 8,10 | s | H 20 |
| 6,60 | s large | NH 8 |
| 6,55 | dd | H 5 |
| 6,15 | d | H 11 |
| 5,80 | dd | H 6 |
| 5,65 | ddd | H 10 |
| 5,50 | d | H 13 |
| 4,95 | ddd | H 14 |
| 4,77 | dd | H 3 |
| 4,75 | d | H 27 |
| 4,27 | ddd | H 24 |
| 4,05 | ddd | H 9 |
| 3,87 | ddd | H 9 |
| 3,80 | s | H 17 |
| 3,55 | ddd | H 24 |
| 3,40 | ddd | H 26 |
| 3,10 | dd système | H 15 |
| 2,9 | dd ABX | H 15 |
| 2,75 | s | $-S-C\underline{H}_2C\underline{H}_2-$ |
| 2,74 | m | H 4 |
| 2,60 | q | $-N-(C\underline{H}_2CH_3)_2$ |
| 2,15 à 1,85 | m | H 25, H 29 |
| 1,70 | s | H 33 |
| 1,05 | m | $-N(CH_2C\underline{H}_3)_2$ + H 32 |
| 0,95 | dd | H 30 + H 31 |

On obtient une solution aqueuse à 2 % de (diéthylamino-2 éthyl) thio-26 pristinamycine $II_B$ (produit BA), à l'état de chlorhydrate, avec:

| | |
|---|---|
| produit BA | 0,1 g |
| acide chlorhydrique 0,05N | 3 $cm^3$ |
| eau distillée ... qsp | 5 $cm^3$ |

**Exemple 2**

En opérant d'une manière analogue à celle décrite à l'exemple 1, mais à partir de 2,7 g de pristinamycine $II_A$ et 0,58 g de diméthylamino-2 éthanethiol et après purification par chromatographie "flash" [éluant: chloroforme-méthanol (90 - 10 en volumes)] et concentration à sec des fractions 11 à 17 sous pression réduite (2,7 kPa) à 30°C, on obtient 1,1 g de (diméthylamino-2 éthyl) thio-26 pristinamycine $II_B$ sous forme d'une poudre jaune fondant vers 100° C.

Spectre RMN:

2,35 (s, 6H : $-N(CH_3)_2$)
2,80 (m, 4H : $-S-C\underline{H}_2C-\underline{H}_2-N$ )
3,40 (ddd, 1H : H 26)
4,75 (d, 1H : H 27)
8,10 (s, 1H : H 20)

On obtient une solution aqueuse à 2 % de (diméthylamino-2 éthyl) thio-26 pristinamycine $II_B$ (produit BB) à l'état de chlorhydrate, avec:

| | |
|---|---|
| produit BB | 0,1 g |
| acide chlorhydrique 0,1N | 1,6 $cm^3$ |
| eau distillée ... qsp | 5 $cm^3$ |

**Exemple 3**

En opérant d'une manière analogue à celle décrite à l'exemple 1, mais à partir de 5,25 g de pristinamycine $II_A$ et 1,3 g de diméthylamino-3 propanethiol et après purification par chromatographie "flash" [éluant : chloroforme-méthanol (90 - 10 en volumes] et concentration à sec des fractions 6 à 29 sous pression réduite (2,7 kPa) à 30°C, on obtient 3,3 g de (diméthylamino-3 propyl) thio-26 pristinamycine $II_B$ sous forme d'une poudre jaune fondant vers 100°C.

Spectre RMN:
1,50 (s, 3H x 0,5 : H 33 1er isomère)
1,70 (s, 3H x 0,5 : H 33 2ème isomère)
1,80 (m, 2H : $-SCH_2-\underline{CH_2}-CH_2N$ )
2,20 (s, 6H x 0,5 : $-N(CH_3)_2$ 1er isomère)
2,25 (s, 6H x 0,5 : $-N(CH_3)_2$ 2ème isomère)
2,40 (m, 2H : $-S\underline{CH_2}-CH_2-CH_2N$ )
2,70 (m, 2H : $-SCH_2-CH_2-\underline{CH_2}N$ )
3,35 } 3,45 } (2m, 1H : H 26 de chaque isomère)
4,60 } 4,70 } (2d 1H : H 27 de chaque isomère)
7,80 } 8,10 } (2s, 1H : H 20 de chaque isomère)

On obtient une solution aqueuse à 3,3 % de (diméthylamino-3 propyl) thio-26 pristinamycine $II_B$ (produit BC), avec:

| | |
|---|---|
| produit BC | 0,1 g |
| acide chlorhydrique 0,1N | 1,55 $cm^3$ |
| eau distillée ... qsp | 3 $cm^3$ |

**Exemple 4**

En opérant d'une manière analogue à celle décrite à l'exemple 1, mais à partir de 5,25 g de pristinamycine $II_A$ et 1,7 g de (pyrrolidinyl-1)-2éthanethiol et après purification par chromatographie "flash" [éluant: chloroforme-méthanol (95 - 5 en volumes)] et concentration à sec des fractions 19 à 60 sous pression réduite (2,7 kPa) à 30°C, on obtient 3,9 g de [(pyrrolidinyl-1)-2 éthyl] thio-26 pristinamycine $II_B$ sous forme d'une poudre jaune fondant vers 115°C.

Spectre RMN:
1,90 (mt, 4H : - 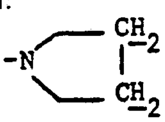 )
2,50 à 2,80 (m, 6H : 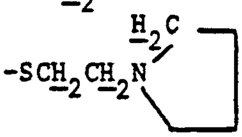 )
3,40 (d, 1H : H 26)
4,75 (d, 1H : H 27)
8,10 (s, 1H : H 20)

On obtient une solution aqueuse à 5 % de [pyrrolidinyl-1)-2 éthyl] thio-26 pristinamycine $II_B$ (produit BD), à l'état de chlorhydrate, avec:

| | |
|---|---|
| produit BD | 0,1 g |
| acide chlorhydrique 0,1N | 1,5 $cm^3$ |
| eau distillée ... qsp | 2 $cm^3$ |

Le (pyrrolidinyl-1)-2 éthanethiol peut être préparé selon la méthode décrite par J.W. HAEFFELE et R.W BROGE, Proc. Sci. Toilet Goods Assoc. 32, 52 (1959) [Chem. Abstr., 54, 17234e (1960)].

**Exemple 5**

En opérant d'une manière analogue à celle décrite à l'exemple 1, mais à partir de 3,15 g de pristinamycine $II_A$ et 1,1 g de (mercapto-2 éthyl)-1 méthyl-4 pipérazine et après purification par chromatographie "flash" [éluant : chloroforme-méthanol (95 - 5 en volumes)] et concentration à sec des fractions 14 à 20 sous pression réduite (2,7 kPa) à 30°C, on obtient 1,4 g de [(méthyl-4 pipérazinyl-1)-2 éthyl] thio-26 pristinamycine $II_B$ sous forme d'une poudre jaune fondant vers 115°C.

Spectre RMN:

2,30 (s, 3H : $>N-CH_3$)
2,40 à 2,80 (m, 12H : 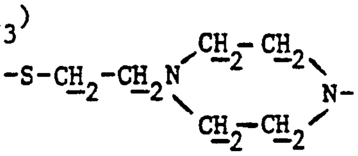 )
3,35 (d, 1H : H 26)
4,75 (d, 1H : H 27)
8,10 (s, 1H : H 20)

On obtient une solution aqueuse à 2 % de [méthyl-4 pipérazinyl-1)-2 éthyl] thio-26 pristinamycine $II_B$ (produit BE), à l'état de chlorhydrate, avec:

| | |
|---|---|
| produit BE | 0,1 g |
| acide chlorhydrique 0,1N | 1,46 $cm^3$ |
| eau distillée ... qsp | 5 $cm^3$ |

La (mercapto-2 éthyl)-1 méthyl-4 pipérazine peut être préparée selon la méthode décrite par D.D. REYNOLDS et coll., J. Org. Chem., 26, 5125 (1961).

**Exemple 6**

En opérant d'une manière analogue à celle décrite à l'exemple 1, mais à partir de 3,15 g de pristinamycine $II_A$ et 1,8 g de diéthylamino-1 propanethiol-2 et après purification par chromatographie "flash" [éluant : chlorure de méthylène-méthanol (90 - 10 en volumes)] et concentration à sec des fractions 3 à 5 sous pression réduite (2,7 kPa) à 30°C, on obtient 1,4 g de (diéthylamino-1 propyl-2) thio-26 pristinamycine $II_B$ sous forme d'une poudre jaune fondant vers 160°C.

Spectre RMN:

1 (m, 9H : H 32 + $-N(CH_2C\underline{H_3})_2$)
2,50 (m, 6H : $-C\underline{H_2}N(C\underline{H_2}CH_3)_2$)
3,30 (m, 1H : H 26)
4,70 (d, 1H : H 27)
8,12 (s, 1H : H 20)

On obtient une solution aqueuse à 5 % de (diéthylamino-1 propyl-2) thio-26 pristinamycine $II_B$ (produit BF) à l'état de chlorhydrate, avec:

| | |
|---|---|
| produit BF | 20 mg |
| acide chlorhydrique 0,1N | 0,3 $cm^3$ |
| eau distillée ... qsp | 0,4 $cm^3$ |

Le diéthylamino-1 propanethiol-2 peut être préparé par la méthode décrite par R.T. WRAGG, J. Chem. Soc. (C), 16, 2087 (1969).

**Exemple 7**

En opérant d'une manière analogue à celle décrite à l'exemple 1, mais à partir de 3,15 g de pristinamycine $II_A$ et 1,6 g de méthyl-1 pipéridinethiol-4 et addition de 0,6 g de triéthylamine au mélange réactionnel et après purification par chromatographie "flash" [éluant : chlorure de méthylène-méthanol (92 - 8 en volumes)] et concentration à sec des fractions 4 à 20 sous pression réduite (2,7 kPa) à 30°C, on obtient 0,9 g de (méthyl-1 pipéridinyl-4) thio-26 pristinamycine $II_B$ sous forme d'une poudre jaune fondant vers 180°C.

Spectre RMN:

2,10 (m, 4H : 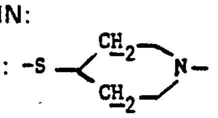 )

2,25 (s, 3H : 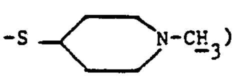)

2,80 (m, 4H : -S 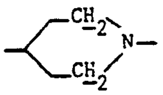 )

3,55 (m, 1H : H 26)
4,62 (m, 1H : H 27)
7,70 (m, 1H : H 8)
8,10 (s, 1H : H 20)

On obtient une solution aqueuse à 5 % de méthyl-1 pipéridinyl-4) thio-26 pristinamycine $II_B$ (produit BG), à l'état de chlorhydrate, avec:

| | |
|---|---|
| produit BG | 10 mg |
| acide chlorhydrique | 0,14 $cm^3$ |
| eau distillée ... qsp | 2 $cm^3$ |

Le méthyl-1 pipéridinethiol-4 peut être préparé par la méthode décrite par H. BARRER et R.E. LYLE, J. Org. Chem., 27, 641 (1962).

**Exemple 8**

En opérant d'une manière analogue à celle décrite à l'exemple 1, mais à partir de 5,25 g de pristinamycine $II_A$ et 10 $cm^3$ d'une solution éthanolique 5N de diméthylamine gazeuse et après purification par chromatographie "flash" [éluant : chloroforme-méthanol (90 - 10 en volumes] et concentration à sec des fractions 14 à 24 sous pression réduite (2,7 kPa) à 30°C, on obtient 0,8 g de diméthylamino-26 pristinamycine $II_B$ sous forme d'une poudre jaune fondant vers 230°C.

Spectre RMN ($CDCl_3$ + 10 % $CD_3OD$)
2,35 (s, 6H : $-N(CH_3)_2$)
3,25 (d, 1H : H 26)
5,05 (d, 1H : H 27)
8,20 (s, 1H : H 20)

On obtient une solution aqueuse à 2 % de diméthylamino-26 pristinamycine $II_B$ (produit BH), à l'état de chlorhydrate, avec:

| | |
|---|---|
| produit BH | 0,1 g |
| acide chlorhydrique 0,1N | 1,75 $cm^3$ |
| eau distillée ... qsp | 5 $cm^3$ |

**Exemple 9**

En opérant d'une manière analogue à celle décrite à l'exemple 1, mais à partir de 5,25 g de pristinamycine $II_4$ et 5 g de méthyl-4 pipérazine et après purification par chromatographie "flash" [éluant : chloroforme-méthanol (90 - 10 en volumes)] et concentration à sec des fractions 20 à 44 sous pression réduite (2,7 kPa) à 30°C, on obtient 0,7 g de (méthyl-4 pipérazinyl-1)-26 pristinamycine $II_B$ sous forme d'une poudre jaune fondant vers 140°C.

Spectre RMN:

2,30 (s, 3H : $>N-CH_3$)

2,40 à 2,65 (m, 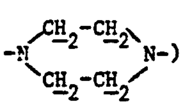 )

3,40 à 3,70 (m contenant H 26)
5,75 (d, 1H : H 27)
8,10 (s, 1H : H 20)

On obtient une solution aqueuse à 3,3 % de (méthyl-4 pipérazinyl-1)-26 pristinamycine $II_B$ (produit BI), à l'état de chlorhydrate, avec:

| | |
|---|---|
| produit BI | 0,1 g |
| acide chlorhydrique 0,1N | 1,6 $cm^3$ |
| eau distillée ... qsp | 3 $cm^3$ |

**Exemple 10**

Une solution de 5,2 g de pristinamycine $II_A$ dans 20 $cm^3$ de méthyl-1 pipérazine est agitée 1 heure 30 minutes à une température voisine de 20° C, puis est versée dans 600 $cm^3$ d'eau distillée. L'émulsion obtenue est extraite 3 fois par 600 $cm^3$ au total de chlorure de méthylène; les phases organiques sont réunies, séchées sur du sulfate de magnésium, filtrées puis concentrées à sec sous pression réduite (2,7 kPa) à 30° C. Le résidu obtenu est purifié par chromatographie "flash" [éluant : chloroforme-méthanol (90 - 10 en volumes)]; après concentration à sec des fractions 13 à 30 sous pression réduite (2,7 kPa) à 30° C, on obtient 2,6 g de (méthyl-4 pipérazinyl-1)-26 pristinamycine $II_B$ sous forme d'une poudre beige fondant vers 140° C.

Le spectre de RMN de ce produit est identique à celui du produit décrit à l'exemple 9.

**Exemple 11**

En opérant d'une manière analogue à celle décrite à l'exemple 1, mais à partir de 12,6 g de pristinamycine $II_A$ et de 5,2 g de bis-diméthylamino-2,3 propanethiol et après purification par chromatographie "flash" [éluant : chlorure de méthylène-méthanol (90 - 10 en volumes)] et concentration à sec des fractions 29 à 42 sous pression réduite (2,7 kPa) à 30° C, on obtient 0,3 g de (bis diméthylamino-2,3 propyl)thio-26 pristinamycine $II_B$ sous forme d'une poudre jaune fondant vers 110° C.

Spectre RMN:

2,30 (m, 12H : 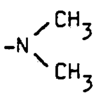 )

2,50 (m, 2H : 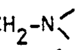 )

2,90 (m, 1H : 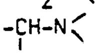 )

3,56 (m, 1H : H26)
4,64 (d, 1H : H27)
4,66 (d, 1H : H27)
7,81 (s, 1H : H20)

On obtient une solution aqueuse à 2 % de (bis diméthyl-amino-2,3 propyl)thio-26 pristinamycine $II_B$ (produit BJ), à l'état de chlorhydrate, avec:

| | |
|---|---|
| produit BJ | 10 mg |
| acide chlorhydrique 0,1N | 0,14 $cm^3$ |
| eau distillée ... qsp | 0,5 $cm^3$ |

Le bis diméthylamino-2,3 propanethiol peut être préparé selon la méthode décrite par H. NISHIMURA et H. TAKAMATSU, Yakugaku Zasshi, 84, 944 (1964) [Chem. Abstr., 62, 2750 b (1965)].

La présente invention concerne également les médicaments constitués par un produit de formule générale I, sous forme libre ou de préférence sous forme de sel d'addition avec un acide pharmaceutiquement acceptable sous forme d'une association avec des synergistines connues ou de préférence avec des synergistines de formule générale V, l'association pouvant en outre contenir tout autre produit pharmaceutiquement compatible, inerte ou physiologiquement actif. Les médicaments selon l'invention peuvent être utilisés par voie parentérale, orale, rectale ou topique.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des sunstances autres que les diluants, par exemple des produits mouillants, édulcorants ou aromatisants.

Les compositions pour administration rectale sont les suppositoires ou les capsules rectales, qui contiennent

outre le principe actif des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les produits selon l'invention associés à des synergistines connues ou de préférence à des synergistines de formule générale V, sont particulièrement utiles dans le traitement des infections d'origine microbienne. Les doses dépendent de l'effet recherché et de la durée du traitement; pour un adulte, elles sont généralement comprises entre 500 et 2000 mg par jour par voie parentérale particulièrement par voie intraveineuse en perfusion lente, la dose de synergistine de formule générale V étant elle-même comprise entre 500 et 2000 mg par jour.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

**Exemple A**

On prépare, selon la technique habituelle, une solution injectable pour perfusion contenant 5 g/l de mélange actif ayant la composition suivante:

| | |
|---|---|
| - (diéthylamino-2 éthyl) thio-26 pristinamycine $II_B$ | 3 g |
| - (diméthylamino-3 propyl) thiométhyl-5 δ pristinamycine $I_A$ | 2 g |
| - solution aqueuse d'acide chlorhydrique 0,1N | 65 $cm^3$ |
| - eau distillée ... qsp | 1000 $cm^3$ |

**Exemple B**

On prépare une solution injectable pour perfusion contenant 1 g/l de mélange actif ayant la composition suivante:

| | |
|---|---|
| - (méthyl-4 pipérazinyl-1)-26 pristinamycine $II_B$ | 0,6 g |
| - [(méthyl-4 pipérazinyl-1)-2 éthyl] thiométhyl-5 δ pristinamycine $I_A$ | 0,4 g |
| - solution aqueuse d'acide chlorhydrique 0,1N | 15,4 $cm^3$ |
| - eau distillée ... qsp | 1000 $cm^3$ |

**Revendications** pour les Etats Contractants: BE, CH, DE, FR, GB, IT, LI, LU, NL, SE

1. Dérivé de la pristinamycine $II_B$, caractérisé en ce qu'il répond à la formule générale I:

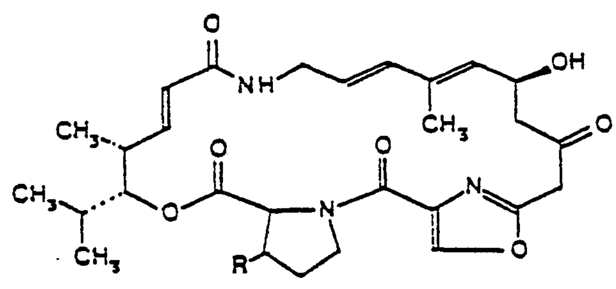

(I)

dans laquelle R représente

- soit un radical alcoylthio substitué:

- i) par un ou deux radicaux alcoylamino ou dialcoylamino dont les parties alcoyle peuvent éventuellement former ensemble avec l'atome d'azote auquel elles sont liées un hétérocycle saturé choisi parmi pyrrolidinyle-1, pipéridino, azétidinyle-1, azépinyle-1, morpholino, thiomorpholino et pipérazinyle-1 (éventuellement substitué par un radical alcoyle), ou bien

- ii) par un radical pyrrolidinyle-2 ou 3, pipéridyle-2, 3 ou 4, azétidinyle-2 ou 3 ou azépinyle-2, 3 ou 4,

- soit un radical de formule générale II:

Het - S - (II)

dans laquelle Het représente un radical pyrrolidinyle-3 pipéridyle-3 ou 4, azétidinyle-3 ou azépinyle-3 ou 4, (éventuellement substitués sur l'atome d'azote par un radical alcoyle)

- soit un radical dialcoylamino dont les parties alcoyle peuvent éventuellement former ensemble avec l'atome d'azote auquel elles sont liées un hétérocycle saturé choisi parmi pyrrolidinyle-1, pipéridino, azétidinyle-1, azépinyle-1, morpholino, thiomorpholino et pipérazinyle-1 (éventuellement substitué par un radical alcoyle), étant entendu que les radicaux et portions alcoyle cités ci-dessus contiennent 1 à 5 atomes de carbone en chaîne droite ou ramifiée, le cas échéant sous ses formes isomères et leurs mélanges, ainsi que ses sels d'addition avec les acides, pharmaceutiquement acceptables.

2. Procédé de préparation d'un dérivé de la pristinamycine $II_B$ selon la revendication 1, caractérisé en ce que l'on fait réagir un produit de formule générale III:

R - H (III)

dans laquelle R est défini comme à la revendication 1 sur la pristinamycine $II_A$ de formule IV:

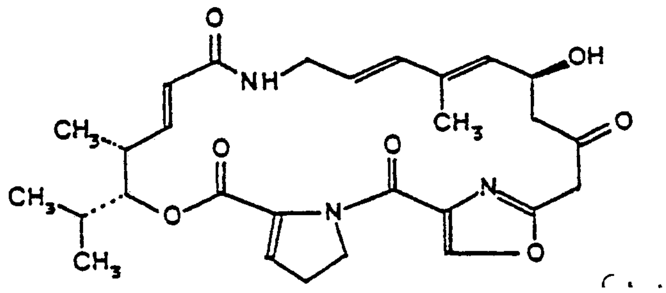

puis le cas échéant sépare les isomères du produit ainsi obtenu et le transforme éventuellement en un sel d'addition avec un acide, pharmaceutiquement acceptable.

3. Composition pharmaceutique caractérisée en ce qu'elle contient au moins un dérivé selon la revendication 1 associé à une synergistine connue ou une synergistine soluble de formule générale V:

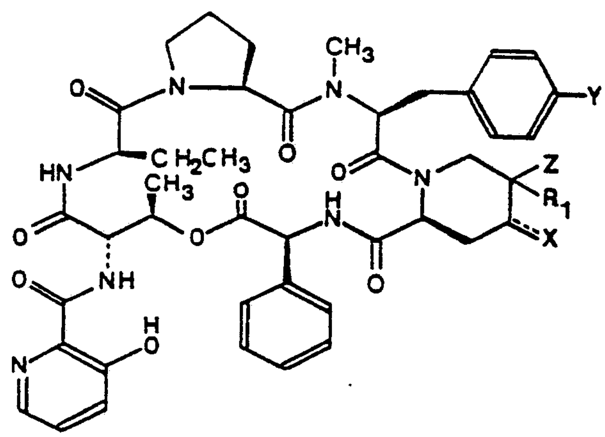

dans laquelle Y représente un atome d'hydrogène ou un radical diméthylamino et

1) ou bien - - - - - représente une simple liaison, Z et $R_1$ représentent un atome d'hydrogène et X représente un radical de formule générale VI:

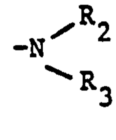

dans laquelle:

- soit $R_2$ représente un atome d'hydrogène et $R_3$ représente un radical hydroxy ou alcoyle éventuellement substitué par un radical carboxy, alcoyloxycarbonyle, hydroxy, alcoylamino ou dialcoylamino dont les radicaux alcoyle peuvent former avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle ou azépinyle, ou bien $R_3$ représente un radical cycloalcoyle contenant 3 à 7 atomes de carbone ou un hétérocycle saturé à 4 à 7

chaînons choisi parmi les cycles azétidine, pyrrolidine, pipéridine et azépine, ces hétérocycles pouvant être éventuellement substitués sur l'atome d'azote par un radical alcoyle,

- soit $R_2$ représente un radical formyle ou alcoylcarbonyle et $R_3$ représente un radical alcoyle substitué par un radical carboxy, alcoylamino ou dialcoylamino dont les radicaux alcoyle peuvent former avec l'atome d'azote auquel ils sont rattachés un hétérocycle à 4 à 7 chaînons choisi parmi azétidinyle, pyrrolidinyle, pipéridinyle, piperazinyle, N-alcoylpipérazinyle ou azépinyle, ou bien $R_3$ représente un hétérocycle à 4 à 7 chaînons choisi parmi les cycles azétidine, pyrrolidine, pipéridine et azépine, ces hétérocycles pouvant être substitués sur l'atome d'azote par un radical alcoyle,

- soit $R_2$ et $R_3$, identiques ou différents, représentent un radical alcoyle éventuellement substitué par un radical carboxy, alcoyloxycarbonyle, hydroxy, alcoylamino ou dialcoylamino dont les radicaux alcoyle forment éventuellement avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle ou azépinyle

- soit $R_2$ et $R_3$ forment ensemble avec l'atome d'azote auquel ils sont rattachés un hétérocycle à 4 à 7 chaînons choisi parmi les cycles azétidine, pyrrolidine, pipéridine, morpholine et pipérazine éventuellement substitué par un radical alcoyle,

2) ou bien ------- représente une double liaison, X représente un atome d'oxygène et Z représente un radical de formule générale VII:

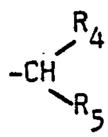

(VII)

défini de la manière suivante:

a) soit $R_1$ et $R_5$ représentent chacun un atome d'hydrogène et $R_4$ représente un radical pyrrolidinyl-3 thio ou pipéridyl-3 ou 4 thio (ces radicaux étant éventuellement substitués par un radical alcoyle) ou bien $R_4$ représente un radical alcoylthio substitué par un ou deux radicaux hydroxysulfonyle, alcoylamino, dialcoylamino (éventuellement substitué par un radical mercapto ou dialcoylamino) ou par un ou deux cycles choisis parmi pipérazino (éventuellement substitué par un radical alcoyle ou mercaptoalcoyle), morpholino, thiomorpholino, pipéridino, pyrrolidinyle-1, pipéridyle-2, 3 ou 4 et pyrrolidinyle-2 ou 3 (ces deux derniers cycles étant éventuellement substitués sur l'atome d'azote par un radical alcoyle),

b) soit $R_1$ et $R_5$ forment ensemble une liaison de valence et $R_4$ représente un radical pyrrolidinyl-3 amino, pipéridyl-3 ou 4 amino, pyrrolidinyl-3 oxy, pipéridyl-3 ou 4 oxy, pyrrolidinyl-3 thio, pipéridyl-3 ou 4 thio (ces radicaux étant éventuellement substitués sur l'atome d'azote du cycle par un radical alcoyle), ou bien $R_4$ représente un radical alcoylamino, alcoyloxy ou alcoylthio substitués par un ou deux radicaux hydroxysulfonyle, alcoylamino, dialcoylamino (éventuellement substitué par un radical dialcoylamino), trialcoylammonio ou imidazolyle-4 ou 5 ou par un ou deux cycles choisis parmi pipérazino (éventuellement substitué par un radical alcoyle ou mercaptoalcoyle), morpholino, thiomorpholino, pipéridino, pyrrolidinyle-1, pipéridyle-2, 3 ou 4 et pyrrolidinyle-2 ou 3 (ces deux derniers cycles étant éventuellement substitués sur l'atome d'azote par un radical alcoyle), étant entendu que les radicaux alcoyle et portions alcoyle se rapportant aux symboles définis ci-dessus

contiennent 1 à 5 atomes de carbone et sont en chaîne droite ou ramifiée, le cas échéant sous forme d'un de ses isomères ou ce leurs mélanges, et éventuellement sous forme de sel d'addition avec un acide, de sel métallique ou de sel d'addition avec une base organique azotée, ladite composition pouvant également contenir d'autres adjuvants pharmaceutiquement acceptables.

**Revendication** pour l'Etat Contractant: AT

Un procédé de préparation de dérivés de la pristinamycine $II_B$ de formule générale I:

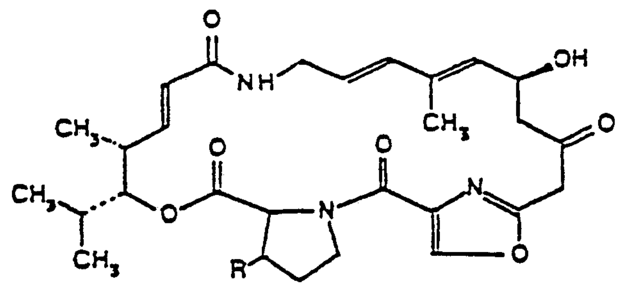

(I)

dans laquelle R représente

- soit un radical alcoylthio substitué:
- i) par un ou deux radicaux alcoylamino ou dialcoylamino dont les parties alcoyle peuvent éventuellement former ensemble avec l'atome d'azote auquel elles sont liées un hétérocycle saturé choisi parmi pyrrolidinyle-1, pipéridino, azétidinyle-1, azépinyle-1, morpholino, thiomorpholino et pipérazinyle-1 (éventuellement substitué par un radical alcoyle), ou bien
- ii) par un radical pyrrolidinyle-2 ou 3, pipéridyle-2, 3 ou 4, azétidinyle-2 ou 3 ou azépinyle-2, 3 ou 4,
- soit un radical de formule générale II:

Het - S - (II)

dans laquelle Het représente un radical pyrrolidinyle-3 pipéridyle-3 ou 4, azétidinyle-3 ou azépinyle-3 ou 4 (éventuellement substitués sur l'atome d'azote par un radical alcoyle)

- soit un radical dialcoylamino dont les parties alcoyle peuvent éventuellement former ensemble avec l'atome d'azote auquel elles sont liées un hétérocycle saturé choisi parmi pyrrolidinyle-1, pipéridino, azétidinyle-1, azépinyle-1, morpholino, thiomorpholino et pipérazinyle-1 (éventuellement substitué par un radical alcoyle), étant entendu que les radicaux et portions alcoyle cités ci-dessus contiennent 1 à 5 atomes de carbone en chaîne droite ou ramifiée, le cas échéant sous leurs formes isomères et leurs mélanges, ainsi que de leurs sels d'addition avec les acides, pharmaceutiquement acceptables, caractérisé en ce que l'on fait agir un produit de formule générale III:

R - H (III)

dans laquelle R est défini comme précédemment sur la pristinamycine $II_A$ de formule IV:

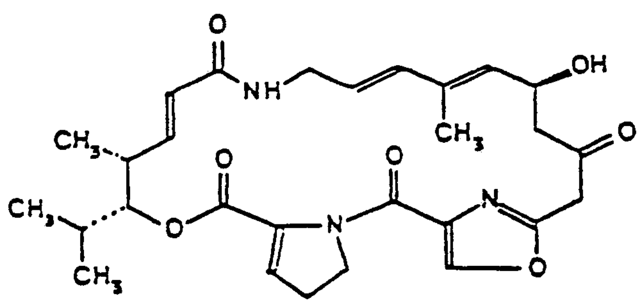

(IV)

puis le cas échéant sépare les isomères du produit ainsi obtenu et le transforme éventuellement en un sel d'addition avec un acide, pharmaceutiquement acceptable.

**Patentansprüche** für die Vertragsstaaten: BE, CH, DE, FR, GB, IT, LI, LU, NL, SE

1. Derivat des Pristinamycins $II_B$, dadurch gekennzeichnet, daß es der allgemeinen Formel (I):

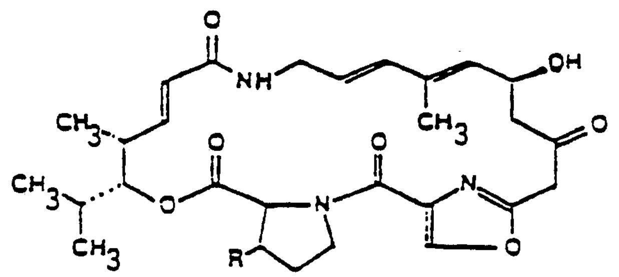

(I)

entspricht, worin R bedeutet

- entweder einen Alkylthiorest, substituiert

- i) durch einen oder zwei Alkylamino- oder Dialkylaminoreste, deren Alkylteile gegebenenfalls zusammen mit dem Stickstoffatom, an das sie gebunden sind, einen gesättigten Heterocyclus bilden können, ausgewählt aus Pyrrolidinyl-1, Piperidino, Azetidinyl-1, Azepinyl-1, Morpholino, Thiomorpholino und Piperazinyl-1 (gegebenenfalls subsitutiert durch einen Alkylrest), oder auch

- ii) durch einen Rest Pyrrolidinyl-2 oder -3, Piperidyl-2, -3 oder -4, Azetidinyl-2 oder -3 oder Azepinyl-2, -3 oder -4,

- oder einen Rest der allgemeinen Formel (II):

Het - S - (II)

worin Het einen Rest Pyrrolidinyl-3, Piperidyl-3 oder -4, Azetidinyl-3 oder Azepinyl-3 oder -4 (gegebenenfalls substituiert am Stickstoffatom durch einen Alkylrest),

- oder einen Dialkylaminorest, dessen Alkylteile gegebenenfalls zusammen mit dem Stickstoffatom, an das sie gebunden sind, einen gesättigten Heterocyclus bilden können, ausgewählt aus Pyrrolidinyl-1, Piperidino, Azetidinyl-1, Azepinyl-1, Morpholino, Thiomorpholino und Piperazinyl-1 (gegebenenfalls substituiert durch einen Alkylrest),

wobei die oben erwähnten Alkylreste und -teile 1 bis 5 Kohlenstoffatome in gerader oder verzweigter Kette enthalten, gegebenenfalls unter seinen isomeren Formen und deren Gemischen sowie seine pharmazeutisch annehmbaren Additionssalze mit Säuren.

2. Verfahren zur Herstellung eines Derivats des Pristinamycins $II_B$ gemäß Anspruch 1, dadurch gekennzeichnet, daß man ein Produkt der allgemeinen Formel (III):

R - H (III)

worin R wie in Anspruch 1 definiert ist, auf das Pristinamycin $II_A$ der Formel (IV):

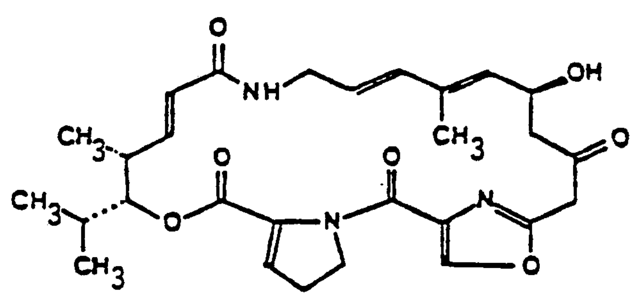

(IV)

einwirken läßt, dann gegebenenfalls die Isomeren des so erhaltenen Produkts trennt und es gegebenenfalls in ein pharmazeutisch annehmbares Additionssalz mit einer Säure überführt.

3. Pharmazeutische Zusammensetzung, dadurch gekennzeichnet, daß sie wenigstens ein Derivat gemäß Anspruch 1 enthält, assoziiert an ein bekanntes Synergistin oder ein lösliches Synergistin der allgemeinen Formel (V):

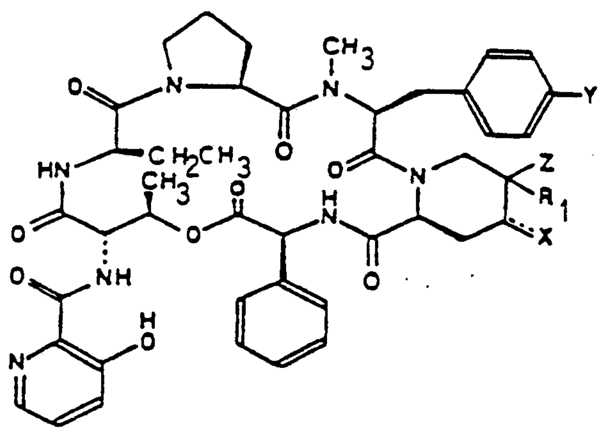

(V)

worin Y ein Wasserstoffatom oder einen Dimethylaminorest bedeutet und

1) entweder ----- eine einfache Bindung bedeutet, Z und $R_1$ ein Wasserstoffatom darstellen und X einen Rest der allgemeinen Formel (VI):

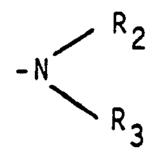

(VI)

bedeutet, worin:

- entweder $R_2$ ein Wasserstoffatom bedeutet und $R_3$ einen Hydroxy- oder Alkylrest bedeutet, gegebenenfalls substituiert durch einen Carboxy-, Alkyloxycarbonyl-, Hydroxy-, Alkylamino- oder Dialkylaminorest, dessen Alkylreste mit dem Stickstoffatom, an das sie gebunden sind, einen Heterocyclus mit 4 bis 7 Kettengliedern bilden können, ausgewählt unter Azetidinyl, Pyrrolidinyl, Piperidinyl, Piperazinyl, N-Alkylpiperazinyl oder Azepinyl, oder $R_3$ bedeutet einen Cycloalkylrest mit 3 bis 7 Kohlenstoffatomen oder einen gesättigten Heterocyclus mit 4 bis 7 Kettengliedern, ausgewählt unter den Ringen Azetidin, Pyrrolidin, Piperidin und Azepin, wobei diese Heterocyclen gegebenenfalls am Stickstoffatom durch einen Alkylrest substituiert sein können,

- oder $R_2$ bedeutet einen Formyl- oder Alkylcarbonylrest und $R_3$ bedeutet einen Alkylrest, substituiert durch einen Carboxy-, Alkylamino- oder Dialkylaminorest, dessen Alkylreste mit dem Stickstoffatom, an das sie gebunden sind, einen Heterocyclus mit 4 bis 7 Kettengliedern bilden können, ausgewählt unter Azetidinyl, Pyrrolidinyl, Piperidinyl, Piperazinyl, N-Alkylpiperazinyl oder Azepinyl, oder $R_3$ bedeutet einen Heterocyclus mit 4 bis 7 Kettengliedern, ausgewählt unter den Ringen Azetidin, Pyrrolidin, Piperidin und Azepin, wobei diese Heterocyclen am Stickstoffatom durch einen Alkylrest substituiert sein können,

- oder $R_2$ und $R_3$, die identisch oder verschieden sind, bedeuten einen Alkylrest, der gegebenenfalls durch einen Carboxy-, Alkyloxycarbonyl-, Hydroxy-, Alkylamino- oder Dialkylaminorest substituiert ist, dessen Alkylreste gegebenenfalls mit dem Stickstoffatom, an das sie gebunden sind, einen Heterocyclus mit 4 bis 7 Kettengliedern bilden, ausgewählt unter Azetidinyl, Pyrrolidinyl, Piperidinyl, Piperazinyl, N-Alkylpiperazinyl oder Azepinyl,

- oder $R_2$ und $R_3$ bilden zusammen mit dem Stickstoffatom, an das sie gebunden sind, einen Heterocyclus mit 4 bis 7 Kettengliedern, ausgewählt unter den Ringen Azetidin, Pyrrolidin, Piperidin, Morpholin und Piperazin, gegebenenfalls substituiert durch einen Alkylrest,

2) oder ------- bedeutet eine Doppelbindung, X bedeutet ein Sauerstoffatom und Z bedeutet einen Rest der allgemeinen Formel (VII):

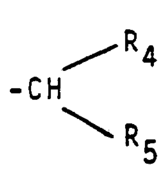

(VII)

der in folgender Weise definiert ist:

a) entweder $R_1$ und $R_5$ bedeuten jeweils ein Wasserstoffatom und $R_4$ bedeutet einen Pyrrolidinyl-3-thio- oder Piperidyl-3- oder -4-thio-rest (diese Reste sind gegebenenfalls substituiert durch einen Alkylrest) oder aber $R_4$ bedeutet einen Alkylthiorest, substituiert durch einen oder zwei Hydroxysulfonyl-, Alkylamino-, Dialkylaminoreste (gegebenenfalls substituiert durch einen Mercapto- oder Dialkylaminorest) oder durch einen oder zwei Cyclen, ausgewählt unter Piperazino (gegebenenfalls substituiert durch einen Alkyl- oder Mercaptoalkylrest), Morpholino, Thiomorpholino, Piperidino, Pyrrolidinyl-1, Piperidyl-2, -3 oder -4 und

Pyrrolidinyl-2 oder -3 (diese beiden letzteren Ringe sind gegebenenfalls am Stickstoffatom durch einen Alkylrest substituiert),

b) oder $R_1$ und $R_5$ bilden zusammen eine Valenzbindung und $R_4$ bedeutet einen Rest Pyrrolidinyl-3-amino, Piperidyl-3- oder -4-amino, Pyrrolidinyl-3-oxy, Piperidyl-3- oder -4-oxy, Pyrrolidinyl-3-thio, Piperidyl-3- oder -4-thio (diese Reste sind gegebenenfalls am Stickstoffatom des Ringes durch einen Alkylrest substituiert) oder aber $R_4$ bedeutet einen Alkylamino-, Alkyloxy- oder Alkylthiorest, substituiert durch einen oder zwei Hydroxysulfonyl-, Alkylamino-, Dialkylaminoreste (gegebenenfalls substituiert durch einen Dialkylaminorest), Trialkylammonio oder Imidazolyl-4 oder -5 oder durch einen oder zwei Ringe, ausgewählt unter Piperazino (gegebenenfalls substituiert durch einen Alkyl- oder Mercaptoalkylrest), Morpholino, Thiomorpholino, Piperidino, Pyrrolidinyl-1, Piperidyl-2, -3 oder -4 und Pyrrolidinyl-2 oder -3 (diese zwei letzteren Cyclen sind gegebebenenfalls am Stickstoffatom durch einen Alkylrest substituiert),

wobei die Alkylreste und Alkylteile, die sich auf die oben definierten Symbole beziehen, 1 bis 5 Kohlenstoffatome enthalten und in gerader oder verzweigter Kette sind, gegebenenfalls in Form eines seiner Isomeren oder ihrer Gemische und gegebenenfalls in Form des Additionssalzes mit einer Säure,des Metallsalzes oder des Additionssalzes mit einer organischen Stickstoffbase, wobei diese Zusammensetzung auch andere pharmazeutisch annehmbare Hilfsmittel enthalten kann.

**Patentanspruch** für den Vertragsstaat: AT

Verfahren zur Herstellung von Derivaten von Pristinamycin $II_B$ der allgemeinen Formel I:

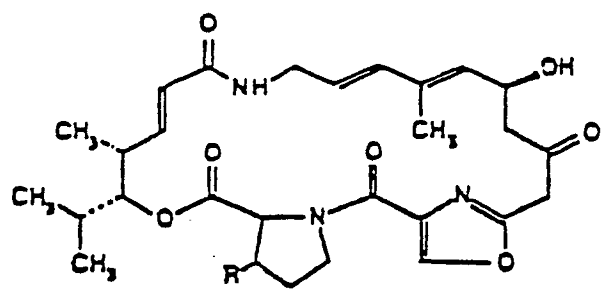

(I)

in welcher R darstellt

- entweder einen Alkylthiorest, substituiert:

- i) durch einen oder zwei Alkylamino- oder Dialkylaminoreste, deren Alkylteile gegebenenfalls zusammen mit dem Stickstoffatom, an das sie gebunden sind, einen gesättigten Heterocyclus bilden können, ausgewählt aus 1-Pyrrolidinyl, Piperidino, 1-Azetidinyl-, 1-Azepinyl, Morpholino, Thiomorpholino und 1-Piperazinyl (gegebenenfalls substituiert durch einen Alkylrest) oder

- ii) durch einen 2- oder 3-Pyrrolidinyl-, 2-, 3- oder 4-Piperidyl-, 2- oder 3-Azetidinyl- oder 2-, 3- oder 4-Azepinylrest,

- oder einen Rest der allgemeinen Formel II:

Het - S - (II)

in welcher Het einen 3-Pyrrolidinyl-, 3- oder 4-Piperidyl-, 3-Azetidinyl- oder 3- oder 4-Azepinylrest (gegebenenfalls substituiert am Stickstoffatom durch einen Alkylrest) darstellt,

- oder einen Dialkylaminorest, dessen Alkylteile gegebenenfalls zusammen mit dem Stickstoffatom, an das sie gebunden sind, einen gesättigten Heterocyclus bilden, ausgewählt aus 1-Pyrrolidinyl, Piperidino, 1-Azetidinyl, 1-Azepinyl, Morpholino, Thiomorpholino und 1-Piperazinyl (gegebenenfalls substituiert durch einen Alkylrest),

wobei die oben genannten Alkylreste und -teile selbstverständlich 1 bis 5 Kohlenstoffatome in gerader oder verzweigter Kette enthalten, gegebenenfalls in ihren isomeren Formen und deren Mischungen, sowie von ihren pharmazeutisch zulässigen Säureadditionssalzen, dadurch gekennzeichnet, da man eine Verbindung der allgemeinen Formel III:

R-H (III),

in welcher R die obige Bedeutung hat, mit dem Pristinamycin $II_A$ der Formel IV:

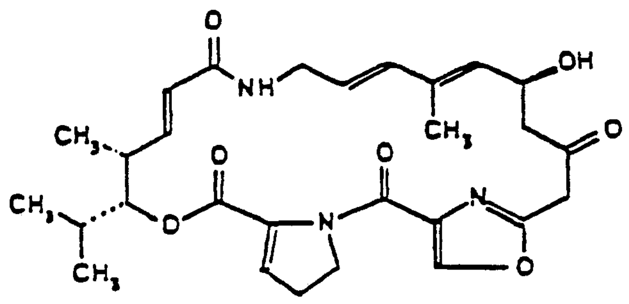

(IV)

umsetzt und dann zutreffendenfalls die Isomeren des so erhaltenen Produktes trennt und es gegebenenfalls in ein pharmazeutisch zulässiges Säureadditionssalz überführt.

**Claims** for the Contracting States: BE, CH, DE, FR, GB, IT, LI, LU, NL, SE

1. Pristinamycin $II_B$ derivative characterized in that it corresponds to the general formula I:

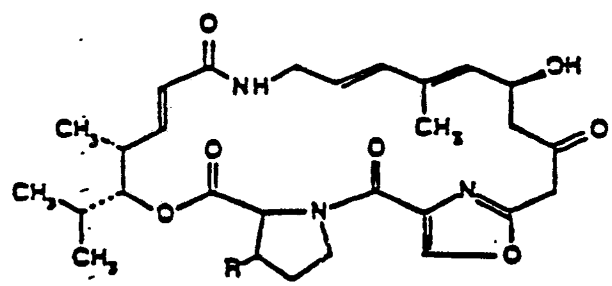

(I)

in which R represents
- either an alkylthio radical substituted:
- i) by one or two alkylamino or dialkylamino radicals of which the alkyl parts can together optionally form, with the nitrogen atom to which they are bonded, a saturated heterocycle chosen from pyrrolidin-1-yl, piperidino, azetidin-1-yl, azepin-1-yl, morpholino, thiomorpholino and piperazin-1-yl (optionally substituted by an alkyl radical), or alternatively
- ii) by a pyrrolidin-2-yl or pyrrolidin-3-yl, piperid-2-yl, piperid-3-yl or piperid-4-yl, azetidin-2-yl or azetidin-3-yl or azepin-2-yl, azepin-3-yl or azepin-4-yl radical, or
- a radical of general formula II:

Het-S- (II)

in which Het represents a pyrrolidin-3-yl, piperid-3-yl or piperid-4-yl, azetidin-3-yl or azepin-3-yl or azepin-4-yl radical (which are optionally substituted on the nitrogen atom by an alkyl radical), or
- a dialkylamino radical of which the alkyl parts can together optionally form, with the nitrogen atom to which they are bonded, a saturated heterocycle chosen from pyrrolidin-1-yl, piperidino, azetidin-1-yl, azepin-1-yl, morpholino, thiomorpholino and piperazin-1-yl (optionally substituted by an alkyl radical), it being understood that the alkyl radicals and alkyl portions mentioned above contain 1 to 5 carbon atoms in a linear or branched chain, if necessary in its isomeric forms and mixtures thereof, and its pharmaceutically acceptable addition salts with acids.

2. Process for the preparation of a pristinamycin $II_B$ derivative according to Claim 1, characterized in reacting a product of the general formula III:

R-H (III)

in which R is defined as in Claim 1, with pristinamycin $II_A$ of formula IV:

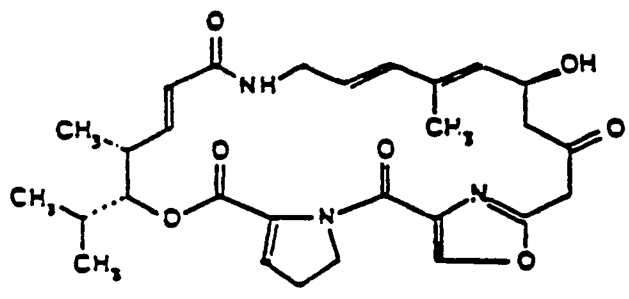

(IV)

and then if necessary separating the isomers of the product thus obtained and, if appropriate, converting it to a pharmaceutically acceptable addition salt with an acid.

3. Pharmaceutical composition characterized in that contains at least one derivative according to Claim 1, in association with a known synergistine or a soluble synergistine of general formula V:

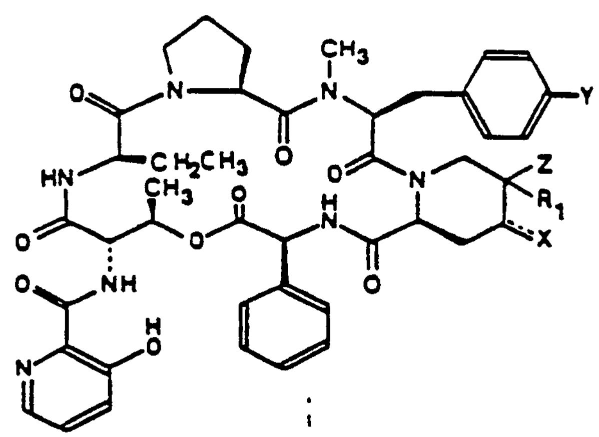

(V)

in which Y represents a hydrogen atom or a dimethylamino radical and

1) either ---- represents a single bond, Z and $R_1$ represent a hydrogen atom and X represents a radical of general formula VI:

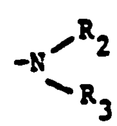

(VI)

in which:

- either $R_2$ represents a hydrogen atom and $R_3$ represents a hydroxyl radical or an alkyl radical optionally substituted by a carboxyl, alkoxycarbonyl or hydroxyl radical or by an alkylamino or dialkylamino radical in which the alkyl radicals can form, with the nitrogen atom to which they are attached, a 4-membered to 7-membered heterocycle, chosen from azetidinyl, pyrrolidinyl, piperidinyl, piperazinyl, N-alkylpiparazinyl, or azepinyl, or alternatively $R_3$ represents a cycloalkyl radical containing 3 to 7 carbon atoms or a saturated 4-membered to 7-membered heterocycle chosen from the azetidine, pyrrolidine, piperidine and azepine rings, it being possible for these heterocycles to be optionally substituted on the nitrogen atom by an alkyl radical,

or

- $R_2$ represents a formyl or alkylcarbonyl radical and $R_3$ represents an alkyl radical substituted by a carboxyl radical or by an alkylamino or dialkylamino radical, the alkyl radicals of which can form, with the nitrogen atom to which they are attached a 4-membered to 7-membered heterocycle chosen from azetidinyl, pyrrolidinyl, piparidinyl, piperazinyl, N-alkylpiperazinyl or azepinyl, or alternatively $R_3$ represents a saturated 4-membered to 7-membered heterocycle chosen from the azetidine, pyrrolidine, piperidine and azepine rings, it being possible for these heterocycles to be substituted on the nitrogen atom by an alkyl radical, or

- $R_2$ and $R_3$, which are identical or different, represent an alkyl radical optionally substituted by a carboxyl, alkoxycarbonyl or hydroxyl radical or by an alkylamino or dialkylamino radical the alkyl radicals of which form, where appropriate, with the nitrogen atom to which they are attached, a 4-membered to 7-membered heterocycle chosen from azetidinyl, pyrrolidinyl, piperidinyl, piperazinyl, N-alkylpiperazinyl or azepinyl, or

- $R_2$ and $R_3$ together form, with the nitrogen atom to which they are attached, a 4-membered to 7-membered heterocycle chosen from the azetidine, pyrrolidine, piperidine, morpholine and piperazine rings optionally substituted by an alkyl radical,

2) or alternatively - - - - - represents a double bond, X represents a oxygen atom and Z represents a radical of general formula VII:

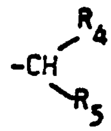

(VII)

defined in the following manner:

a) either $R_1$ and $R_5$ each represent a hydrogen atom and $R_4$ represents a pyrrolidin-3-ylthio or piperid-3-ylthio or piperid-4-ylthio radical (these radicals being optionally substituted by an alkyl radical), or alternatively $R_4$ represents an alkylthio radical substituted by one or two hydroxysulphonyl, alkylamino or dialkylamino (optionally substituted by a mercapto or a dialkylamino radical) radicals or by one or two rings chosen from piperazino (optionally substituted by an alkyl or mercaptoalkyl radical), morpholino, thiomorpholino, piperidino, pyrrolidin-1-yl, piperid-2-yl, -3-yl or -4-yl and pyrrolidin-2-yl or -3-yl (these two latter rings being optionally substituted on the nitrogen atom by an alkyl radical), or

b) $R_1$ and $R_5$ together form a valency bond and $R_4$ represents a pyrrolidin-3-ylamino, piperid-3-ylamino or piperid-4-ylamino, pyrrolidin-3-yloxy, piperid-3-yloxy or piperid-4-yloxy, pyrrolidin-3-ylthio, piperid-3-ylthio or piperid-4-ylthio radical (these radicals being optionally substituted on the nitrogen atom of the ring by an alkyl radical), or alternatively $R_4$ represents an alkylamino, alkoxy or alkylthio radical which are substituted by one or two hydroxysulphonyl, alkylamino or dialkylamino (optionally substituted by a dialkylamino radical) radicals or trialkylammonio or imidazol-4-yl or imidazol-5-yl radicals or by one or two rings chosen from piperazino (optionally substituted by an alkyl or mercaptoalkyl radical), morpholino, thiomorpholino, piperidino, pyrrolidin-1-yl, piperid-2-yl, -3-yl or -4-yl and pyrrolidin-2-yl or -3-yl (these two latter rings being optionally substituted on the nitrogen atom by an alkyl radical), it being understood that the alkyl radicals and alkyl portions relating to the symbols defined above contain 1 to 5 carbon atoms and are in a linear or branched chain, if necessary in the form of one if its isomers or of their mixtures, and optionally in the form of addition salt with an acid, of metal salt or of addition salt with a nitrogenous organic base, it also being possible for the said composition to contain other pharmaceutically acceptable adjuvants.

**Claim** for the Contracting State: AT

A process for the preparation of pristinamycin $II_B$ derivatives of general formula I:

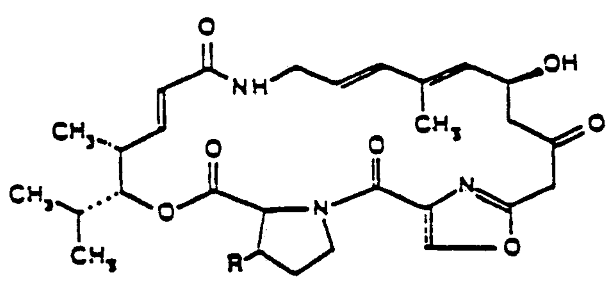

(I)

in which R represents

- either an alkylthio radical substituted:
- i) by one or two alkylamino or dialkylamino radicals of which the alkyl parts can together optionally form, with the nitrogen atom to which they are bonded, a saturated heterocycle chosen from pyrrolidin-1-yl, piparidino, azetidin-1-yl, azepin-1-yl, morpholino, thiomorpholino and piperazin-1-yl (optionally substituted by an alkyl radical), or alternatively
- ii) by a pyrrolidin-2-yl or pyrrolidin-3-yl, piperid-2-yl, piperid-3-yl or piperid-4-yl, azetidin-2-yl or azetidin-3-yl or azepin-2-yl, azepin-3-yl or azepin-4-yl radical, or
- a radical of the general formula II:

Het-S- (II)

in which Het represents a pyrrolidin-3-yl, piperid-3-yl or piperid-4-yl, azetidin-3-yl or azepin-3-yl or azepin-4-yl radical (which are optionally substituted on the nitrogen atom by an alkyl radical), or

- a dialkylamino radical of which the alkyl parts can together optionally form, with the nitrogen atom to which they are bonded, a saturated heterocycle chosen from pyrrolidin-1-yl, piperidino, azetidin-1-yl, azepin-1-yl, morpholino, thiomorpholino and piperazin-1-yl (optionally substituted by an alkyl radical), it being understood that the alkyl radicals and portions mentioned above contain 1 to 3 carbon atoms in a linear or branched chain, if necessary in their isomeric forms and mixtures thereof, and their pharmaceutically acceptable addition salts with acids, characterized in reacting a product of general formula III:

R-H (III)

in which R is defined as above, with pristinamycin $II_A$ of formula (IV):

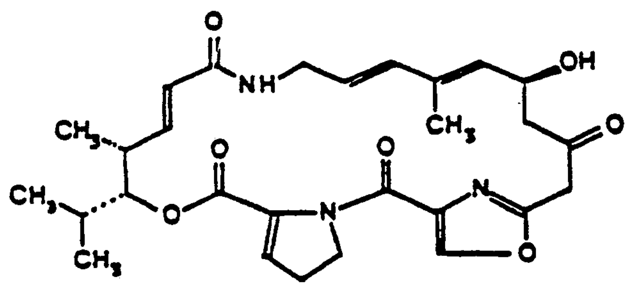

(IV)

and then if necessary separating the isomers of the product thus obtained and, if appropriate, converting it to a pharmaceutically acceptable addition salt with an acid.